# FASCICULE DE BREVET EUROPEEN

(11) **EP 0 748 617 B1**
(45) Date de publication et mention de la délivrance du brevet: **18.12.2002**
(21) Numéro de dépôt: 96401214.0
(22) Date de dépôt: 06.06.1996
(51) Int. Cl.: A61F 9/00, B05B 11/00

(54) **Distributeur double de liquides médicamenteux**
Doppelfluidabgabeeinrichtung für Wirkstoffflüssigkeiten
Double dispenser for medicating fluids

(30) Priorité: 14.06.1995 FR 9507087
(43) Date de publication de la demande: 18.12.1996
(73) Titulaire: Py, Daniel, 78100 Saint-Germain-en-Laye (FR)
(72) Inventeur: Py, Daniel, 78100 Saint-Germain-en-Laye (FR)
(74) Mandataire: Rinuy, Santarelli

(56) Documents cités:
- WO-A-93/19806
- WO-A-94/03135
- WO-A-94/20408
- FR-A- 2 725 247
- US-A- 5 320 845

## Description

La présente invention concerne un distributeur double de liquides médicamenteux.

L'instillation topique est la route d'administration la plus commune pour les médicaments ophtalmiques depuis que les traitements oculaires existent. C'est pourquoi on s'attendrait à ce qu'elle soit simple, pratique et efficace.

Bien qu'habituellement un certain nombre de patients puissent s'administrer eux-mêmes leurs médicaments, les études d'observance des prescriptions médicamenteuses montrent qu'un grand nombre d'entre eux n'y parviennent pas. La nécessité en médecine ambulatoire de se reposer sur le malade pour effectuer un traitement correct est un facteur de non observation des prescriptions des médecins et très spécialement lorsque le traitement est aussi délicat qu'en ophtalmologie.

L'impossibilité pour un patient de localiser l'extrémité des comptes-gouttes habituels sans miroir ou sans verres correcteurs lorsque ceux-ci sont nécessaires pour se regarder dans un miroir, est très dangereuse. Il y a un risque important de blessures de la cornée ainsi qu'un risque de contamination du principe actif du médicament si l'extrémité du compte-goutte entre en contact avec la conjonctive comme dans la majorité des cas.

De plus, malgré les difficultés ci-dessus, lorsqu'une goutte est administrée avec succès à l'oeil, une perte significative de médicament intervient par débordement sur le visage et par drainage dans le canal lacrymo-nasal.

La diffusion de la substance active dans la circulation générale intervient essentiellement par l'intermédiaire du conduit lacrymo-nasal. Sa présence dans la circulation systémique contribue à l'apparition d'effets secondaires généraux graves suite à une simple instillation oculaire et ceci a créé un intérêt considérable pour la conception de divers systèmes d'administration de principes actifs qui pourraient minimiser le risque d'une toxicité systémique.

Si l'on inclut le liquide capturé dans les plis de la conjonctive, le volume normal des larmes dans l'oeil est d'environ 10 ml. Un volume total de liquide d'environ 20 ml peut être gardé un bref instant, si les paupières ne sont pas refermées à la suite de l'administration du médicament topique. Lorsqu'une seule goutte de médicament est appliquée à l'oeil, habituellement d'un volume de 30 à 60 ml, la plus grande partie de la goutte administrée coule sur les joues tandis que le système de drainage lacrymo-nasal draine rapidement le volume excédentaire.

Ainsi, l'augmentation de la taille des gouttes n'augmente pas la biodisponibilité. Selon certains auteurs, 5 % seulement de la quantité totale des ingrédients actifs atteint en fait l'humeur aqueuse.

A l'inverse, la taille importante des gouttes provoque une exacerbation de la sensibilité cornéenne qui, elle-même, génère deux réflexes synergiques de défense : le clignement des yeux et le larmoiement, la combinaison des deux conduisant à l'élimination de la plus grande partie de la goutte ophtalmique.

Cependant, l'absorption systémique est accrue proportionnellement à la taille de la goutte, étant donné qu'ensuite, le drainage au travers du canal lacrymo-nasal, conduit habituellement le principe actif à être absorbé dans le plasma par la muqueuse nasale voire être dégluti.

Lorsque l'on utilise, en ophtalmologie, des principes actifs doués d'importants effets secondaires systémiques potentiels, tels les bêta-bloquants, il est important d'essayer de limiter la taille des gouttes pour minimiser les risques d'effets secondaires. En vue de réduire l'absorption systémique, on a par exemple proposé la compression de l'extrémité nasale de la paupière inférieure pendant cinq minutes après l'instillation pour provoquer l'occlusion du point lacrymal. C'est pourquoi il serait souhaitable de disposer d'un appareil conçu pour prévenir les résorptions systémiques, c'est à dire pour délivrer une dose de produit(s) actif(s) dont le volume n'excède pas le volume disponible dans l'espace conjonctival de 25 ml maximum.

Certaines pathologies nécessitent, dans un bon nombre de cas, l'association de deux principes actifs. Il serait souhaitable que ledit appareil permette de délivrer par exemple deux gouttes séparées de 12,5 ml de deux liquides différents. Bon nombre des principes actifs à usage ophtalmique sont très difficiles à combiner dans une même formulation. Il en est ainsi par exemple lorsque l'on veut associer un principe actif hydrophile avec un principe actif lipophile. Un telle combinaison est irréalisable dans le même flacon sans changement important, d'au moins une formulation, c'est à dire sans réduction importante de sa pénétration ou de son activité. De plus, administrer deux gouttes conventionnelles séparées conduit à des volumes excessifs et à encore plus d'effets secondaires et moins de produits actifs dans l'oeil puisque la deuxième goutte lave la première.

C'est par exemple le cas d'une combinaison de dorzolamide (Trusopt) et d'épinephrine. Les volumes respectifs administrés de chacun de ces deux composants sont habituellement de 30 ml pour le premier et 40 à 60 ml pour le second, ce qui conduit à un volume total minimum d'environ 70 ml. Or, comme on l'a vu ci-dessus, la capacité de l'oeil est au maximum de 20 à 25 ml. C'est pourquoi une formule combinée de cette association a été développée par le fabricant, au prix de difficultés majeures, d'un budget énorme et plus de douze ans de développement.

De plus, il serait souhaitable que l'administration du médicament intervienne en une partie de l'oeil peu sensible afin d'éviter les réactions ci-dessus mentionnées. Une telle zone peu sensible est le cul de sac conjonctival inférieur.

Il serait donc souhaitable de disposer d'un dispositif d'instillation capable de délivrer simultanément deux principes actifs séparés, répondant aux objectifs et contraintes définis ci-dessus.

Un tel dispositif devrait, de préférence, être pratique, permettant à un patient de s'instiller facilement et de manière concomitante deux gouttes séparées d'une médication par auto-administration, dans n'importe quel environnement, que ce soit à la maison, au travail ou ailleurs, sans avoir à basculer la tête en arrière.

Ce dispositif devrait être sûr, éliminer les risques actuels d'endommager l'oeil et de contaminer les contenus.

En outre, la taille des gouttes ainsi administrées devrait être faible, de manière à ce que la somme des deux gouttes administrées n'excède pas la capacité maximale de l'oeil, afin de contrôler la dose de médicament effectivement administrée, et minimiser le risque d'absorption systémique avec la toxicité potentielle qui l'accompagne.

Dans d'autres domaines que l'ophtalmologie, certaines pathologies nécessitent aussi, dans un bon nombre de cas, l'association de deux principes actifs. Bon nombre des principes actifs par exemple en Oto-Rhino-Laryngologie, Pneumologie, Dermatologie, Gynécologie sont très difficiles à combiner dans une même formulation. Il en est ainsi par exemple comme on l'a mentionné ci-dessus, lorsque l'on veut associer en milieu liquide un principe actif hydrophile avec un principe actif lipophile ou encore deux principes actifs dont les pH de pénétration et d'activité sont différents l'un de l'autre. Une telle combinaison est irréalisable dans le même flacon sans changement important, d'au moins une formulation, c'est à dire sans réduction importante de sa pénétration ou de son activité.

C'est pourquoi la présente demande a pour objet un distributeur double de liquides médicamenteux, caractérisé en ce qu'il comprend :
- un carter extérieur comprenant au moins une ouverture avant et une ouverture arrière,
- ledit carter contenant deux pompes individuelles à déclenchement de jet jumelé, munies chacune d'un dispositif doseur délivrant des doses déterminées, la direction et le sens du jet projeté par lesdites pompes par l'ouverture avant du carter convergeant vers un point situé au delà de l'extrémité avant du carter selon un angle tel que l'écartement des jets soit prédéterminé, les deux jets quittant le distributeur par deux orifices d'éjection distincts,
- un dispositif de déclenchement des jets, accessible depuis l'extérieur du carter.

En vue particulièrement d'applications ophtalmiques, un distributeur de liquides médicamenteux préféré selon l'invention est caractérisé en ce qu'il comprend un doigt proéminent à l'avant du carter. Dans un tel cas notamment, on préfère un distributeur caractérisé en ce que la direction et le sens du jet projeté par lesdites pompes par l'ouverture avant du carter convergent vers un point situé au delà de l'extrémité du doigt écartée du carter, et au dessus de celui-ci selon un angle tel qu'au niveau de ladite extrémité, l'écartement des jets soit d'environ 3 à 5 mm.

Le doigt peut avoir par exemple une longueur de 1 à 4 cm, mais de préférence d'environ 2 cm ; sa largeur pourra aller par exemple de 2 à 30 mm, mais de préférence de 8 à 15 mm. Il pourra être fixe, mais de préférence rétractable, repliable, ou encore amovible.

Ce doigt lorsqu'il est destiné à venir en contact avec la base de la paupière inférieure, et à tirer celle-ci vers le bas, sera fait ou recouvert avantageusement d'une matière souple, non agressive, et de préférence antidérapante, telle une matière caoutchoutée naturelle comme le latex, ou synthétique comme un élastomère. Le doigt est situé à l'avant du dispositif, l'avant étant par définition le côté par lequel sort un liquide oculaire pour être administré. Le doigt peut avoir diverses formes selon l'utilisation envisagée, tenant compte du fait que sa fonction est d'écarter le distributeur du lieu d'application des principes actifs.

Les pompes individuelles sont de tout type connu notamment du type à accordéon ou à piston, de préférence du type nécessitant un armement préalable, et par exemple du type décrit dans le brevet US N° 5 267 986, capables d'éjecter un volume prédéterminé. Une pompe unitaire, telle que celle décrite, en configuration double, dans le brevet US N° 5 320 845 et permettant l'administration si désiré d'un volume de 10 ml environ est également utilisable, bien que ne disposant pas d'un dispositif d'armement préalable. En effet, une telle pompe permet la projection d'un principe actif sans conservateur. Un autre type de pompe est aussi décrit dans le brevet WO-A-9420408. Une pompe adaptée est également, et de préférence, une pompe du type décrit dans FR-A-2725247 (cité selon l'article 54(3) CBE).

De telles pompes conviennent particulièrement à des préparations stériles tels des liquides ou des gels ophtalmiques. Dans ce qui suit le terme "liquide" vise indifféremment l'une ou l'autre consistance, sauf exception signalée.

Les pompes sont armées de préférence par pression à l'arrière du carter, si l'on considère que la partie avant du dispositif selon l'invention est celle par laquelle sortent les jets ou comportant le doigt, et que donc le doigt se trouve en conséquence à la partie inférieure du carter. Elles peuvent aussi être armées latéralement.

Elles peuvent être armées simultanément ou séparément, le déclenchement des jets étant par contre simultané. De ce fait, elles sont munies d'un dispositif commun de déclenchement. Bien évidemment, si une seule des pompes est armée, un seul jet sera déclenché. A titre de sécurité, si l'on veut imposer un déclenchement des deux jets, on peut prévoir que les deux pompes seront nécessairement armées ensemble, par exemple à l'aide d'un dispositif de jumelage d'armement.

En vue de leur armement et de leur déclenchement, les pompes sont avantageusement contenues dans des boîtiers rigides, comportant notamment des encoches latérales.

Les pompes unitaires utilisées auront de préférence, à chaque déclenchement, un volume d'éjection inférieur à 20 ml, généralement inférieur à 15 ml, plus particulièrement de 7 à 12,5 ml, la somme des volumes éjectés étant de préférence inférieure à environ 30 ml et notamment inférieure à environ 25 ml. Mais de petites doses peuvent encore être administrées en particulier en utilisant une pompe telle que décrite dans FR-A-2725247 ainsi que ci-après.

Compte tenu de l'installation particulière des pompes dans leur carter, lorsque l'on applique le doigt à la base d'une paupière, et on descend celle-ci vers le bas pour dévoiler le cul de sac conjonctival inférieur, lors du déclenchement des pompes, le liquide est éjecté dans ce cul de sac, en deux jets écartés de quelques millimètres l'un de l'autre, de préférence environ à 10 mm.

Selon l'invention, la tête des pompes est orientée de telle sorte que leurs jets convergent. La totalité de la pompe peut avoir une telle orientation, dans lequel cas, les pompes forment un "V". Dans une autre forme de réalisation, seules les têtes des pompes peuvent avoir cette orientation, les corps des pompes étant parallèles, ce qui permet un gain sur la largeur maximale du dispositif.

Le dispositif de déclenchement des pompes provoquant l'éjection du liquide ophtalmique est de tout type connu, à gâchette, bouton-poussoir .... Il peut être séparé sur chaque pompe, mais, pour les raisons évoquées précédemment, ce dispositif est avantageusement d'action simultanée sur les deux pompes. Il peut être situé notamment au-dessus ou au-dessous du carter et de préférence au-dessus pour que, par exemple, le mouvement du doigt dans le cas d'un bouton poussoir, contribue à abaisser la paupière en même temps.

Dans des conditions préférentielles de réalisation, le dispositif de déclenchement des jets est un bouton-poussoir accessible depuis l'extérieur du carter et comportant deux languettes libres à leur extrémité opposée au bouton, de préférence parallèles, munies de deux ailes perpendiculaires à ces languettes. Ces ailes peuvent alors coopérer à leur extrémité libre pour déclencher les jets, avec une fente en forme de V ménagée sur la paroi opposée du carter pour provoquer le rapprochement des languettes et le désengagement des ailes des encoches latérales ménagées dans des boîtiers rigides renfermant les pompes.

Dans des conditions de réalisation tout particulièrement préférées d'un distributeur selon l'invention, les pompes comprennent trois pièces, à savoir un corps de pompe, un piston et un flacon élastique les renfermant, le corps de pompe ayant une extrémité antérieure du côté de la sortie de la pompe, ladite extrémité antérieure comportant un orifice de sortie obturé par une membrane élastique constituant l'avant du flacon et se poursuivant vers l'arrière par un conduit de pompe muni d'un orifice latéral d'admission de fluide, le piston étant installé dans ledit corps de pompe, le déplacement relatif de l'extrémité du piston par rapport au corps de pompe entre l'orifice d'admission et une position en butée située vers l'orifice de sortie déterminant ainsi la quantité de fluide expulsé lors du déplacement relatif piston/corps de pompe.

Dans d'autres conditions préférentielles, le flacon comprend une première partie en accordéon d'une épaisseur suffisante pour former un moyen de rappel acceptable et une seconde partie en accordéon, le piston comprenant un anneau postérieur bloqué entre les deux parties en accordéon, et le corps de pompe étant cylindrique et comprenant un anneau frontal, solidaire de l'avant du flacon, ainsi qu'un anneau postérieur, solidaire de l'arrière du flacon derrière la partie en accordéon.

Dans un dispositif selon l'invention, le doigt permet en ophtalmologie à la fois d'abaisser la paupière et d'exposer une zone peu sensible et douée d'une bonne capacité d'absorption à savoir le cul de sac conjonctival inférieur normalement caché derrière la paupière inférieure, et de plus il permet d'empêcher l'extrémité du flacon dans lequel est effectivement contenu le produit de toucher l'oeil et ainsi évite toute contamination de ce récipient.

En raison de son action combinée synergique avec le doigt comme décrit ci-dessus, l'application sur le sourcil de la partie supérieure du carter, permet à l'axe principal du dispositif selon l'invention d'être aligné avec le cul de sac conjonctival mis en évidence et ainsi permet à deux jets séparés d'être propulsés exactement dans le cul de sac conjonctival ainsi dévoilé. C'est pourquoi de préférence, la partie supérieure avant du carter a une forme courbe, épousant l'arcade sourcilière et ladite partie supérieure du carter, l'extrémité du doigt, et les axes de projection des pompes sont agencés pour que les jets soient dirigés de préférence vers le cul de sac conjonctival par traction vers le bas de la paupière inférieure, et écartés de 2 à 6 mm au niveau du point d'impact des jets sur la muqueuse du cul de sac conjonctival. Bien évidemment, d'autres conformations permettant une bonne visée sont réalisables. Au lieu de mettre la partie supérieure du carter en contact avec le sourcil, le patient peut également interposer un doigt, ou encore un capuchon articulé pour dévoiler les buses des pompes et c'est ce capuchon en position d'ouverture que l'on applique sur les sourcils.

Les pompes qui, compte-tenu du volume éjecté peuvent être appelées micro-pompes, projettent leur contenu respectif dans le cul de sac éversé. Ceci permet,
- d'une part, en raison du faible volume des gouttes combinées délivrées par les pompes, d'éviter le clignement des yeux, le larmoiement et le débordement sur les joues.
- d'autre part, étant donné que le liquide est projeté, il n'est pas nécessaire de rejeter la tête vers l'arrière.

Un volume de goutte de liquide ophtalmique prédéterminé et constant est ainsi obtenu : pour la première fois en ophtalmologie, un volume de liquide d'environ 15 ml ou inférieur, permet d'assurer une meilleure pénétration oculaire conduisant ainsi à une efficacité accrue, la suppression de la majeure partie de la résorption systémique, et la délivrance efficace de deux gouttes simultanément dans l'oeil sans débordement hors de l'oeil ou par les voies lacrymales.

Les pompes utilisées sont avantageusement des pompes munies d'une valve anti-retour.

L'utilisation d'un carter et d'un dispositif unique de déclenchement, combiné à un armement des pompes, permet l'administration du liquide par une simple pression sur le déclencheur, après que le patient a descendu vers le bas sa paupière inférieure grâce au doigt.

Le dispositif selon la présente invention procure au patient de nombreux avantages. Ainsi il n'a plus à s'administrer deux gouttes différentes à plusieurs minutes d'intervalle avec le risque d'oublier la seconde. Puisque le liquide est expulsé par les pompes, il n'a plus besoin, en ophtalmologie, de rejeter la tête en arrière pour s'administrer le produit.

L'administration en ophtalmologie ne provoque plus de larmoiement ni de débordement de liquide sur les joues.

Le patient n'a pas d'inconfort dans son traitement puisque le point en ophtalmologie où sont administrés les liquides est un emplacement de faible sensibilité, il n'a plus besoin de miroir ou d'une autre personne pour lui administrer son médicament.

Les améliorations sur le plan de la pharmacologie sont également remarquables. Une meilleure pénétration oculaire est assurée par la petite taille des gouttes propulsées dans le cul de sac conjonctival, qui ne stimulent pas les nerfs sensitifs de la cornée. De ce fait, il n'y a pas de clignement des yeux ni de réflexe de larmoiement pouvant laver le principe actif et l'éliminer de cette zone de faible sensibilité.

Le cul de sac conjonctival où sont administrés les produits est une zone de faible renouvellement de larmes, étant donné qu'habituellement la paupière inférieure ne cligne pas. La délivrance du liquide dans cette zone spécifique augmente le temps de résidence du principe actif dans le cul de sac conjonctival.

Deux micro-gouttes séparées et simultanément administrées, spécifiquement dans le cul de sac conjonctival sont aussi plus efficaces qu'une formulation combinée de 30 à 60 ml de deux différents liquides ophtalmiques classiques, tant par le respect des pH spécifiques aux deux formulations que par le volume total administré impossible à obtenir par les seules pesanteur et tension superficielle des instillateurs actuels.

La délivrance de petites gouttes dans le cul de sac conjonctival n'expose pas la cornée à un possible contact avec les principes actifs.

Les compte-gouttes de l'art antérieur délivrant un volume de liquide ophtalmique supérieur au volume maximum que le segment antérieur de l'oeil peut accepter provoquent au moins une immersion partielle de la cornée, tissu le plus sensible du corps humain.

Etant donné que les gouttes administrées avec le dispositif selon la présente invention sont délivrées dans le cul de sac conjonctival à faible sensibilité et à faible larmoiement, même si le pH de la formulation du principe actif est agressif pour la cornée, la tolérance à ces principes actifs qui provoquent les yeux rouges doit être significativement améliorée.

Le dispositif selon l'invention présente également d'importants avantages économiques. Il permet de réduire de façon très importante le temps nécessaire pour obtenir l'autorisation de mise sur le marché d'une association de principes actifs différents, par rapport au temps nécessaire pour obtenir l'autorisation de commercialiser une formulation combinée ; en effet, la durée et le coût de développement d'une association sont comparables à ceux nécessaires au développement d'un principe actif unique original voire plus.

De plus un tel dispositif rend la réalisation d'une combinaison de deux principes actifs différents en une seule formulation pour utilisation ophtalmique, complètement obsolète. Les galénistes n'auront, en particulier plus à longuement étudier quelles formulations particulières pourraient permettre de combiner dans un même récipient deux principes actifs dont les plages de pH d'activité peuvent être différentes ou pouvant avoir des solubilités différentes notamment si l'un d'entre eux est hydrophile et l'autre lipophile.

L'utilisation d'un système à clapet anti-retour pour les pompes permet la délivrance de formulations sans conservateurs, même en gel. Le dispositif selon l'invention est le seul système qui actuellement permette de délivrer des doses prédéterminées avec une variation inférieure à 10 %.

Le dispositif selon l'invention est également extrêmement sûr :

Les effets combinés des deux principes actifs différents seront réellement obtenus, et ce sans avoir à répéter deux administrations différentes espacées de quelques minutes.

Le dispositif selon l'invention permet également d'accroître l'index thérapeutique, absorption topique / absorption systémique, à la fois en réduisant la résorption systémique par le canal lacrymal et en augmentant l'absorption topique en ophtalmologie.

L'utilisation d'un doigt souple de taille appropriée permet d'éviter tout traumatisme de la cornée.

Lorsque le dispositif est utilisé correctement, c'est à dire avec appui de la partie supérieure du carter, directement ou par interposition d'un doigt du patient sur ses sourcils et le doigt du dispositif tirant la paupière inférieure vers le bas, les gouttes de liquide ophtalmique sont propulsées exactement dans le cul de sac conjonctival sans le moindre contact entre l'extrémité des pompes et la conjonctive ou la cornée.

Si l'on utilise des pompes à valve anti-retour, aucune contamination du contenu des pompes et des flacons n'est possible.

L'utilisation d'un capot articulé permet d'éviter qu'un capuchon ne tombe sur le sol ou sur quelque surface septique avant d'être remis en place comme pour les flacons conventionnels, en contaminant le contenu du flacon.

L'invention a enfin pour objet un déclencheur caractérisé en ce qu'il comprend un bouton poussoir comportant au moins une languette libre à son extrémité opposée au bouton, munie d'une aile perpendiculaire à ladite languette, capable de coopérer à son extrémité libre avec la paroi inclinée d'une cuvette, une pression sur le bouton provoquant ainsi une déplacement de l'aile perpendiculairement à la direction d'enfoncement du bouton poussoir.

Si l'aile, en position non enfoncée du poussoir, est introduite dans la rainure d'un élément sous tension mécanique (tel que le boîtier d'une pompe ci-dessus), lorsque l'on appuie sur le bouton-poussoir, l'aile se dérobe et relâche ledit élément qui peut ainsi se déplacer longitudinalement.

Le déclencheur ci-dessus peut comprendre deux languettes et ailes, ou plus. Dans le cas de deux languettes, la cuvette sera allongée en forme de V. Dans le cas de trois languettes ou plus, la cuvette pourra par exemple être conique.

De préférence l'élasticité des languettes et leur rigidité est suffisante pour que le bouton-poussoir remonte de lui-même en position non appuyée. On peut aider cette remontée par exemple à l'aide d'un élément élastique tel un ressort situé entre le bouton et le fond de la cuvette, du moment que l'on préserve un débattement suffisant des ailes vers ledit ressort pour permettre l'escamotage des ailes.

L'invention sera mieux comprise si l'on se réfère aux dessins annexés dans lesquels :

La figure 1 représente un distributeur oculaire double selon l'invention vu de dessus, pompes au repos.

La figure 2 est une vue analogue mais vue de côté, le capot avant de protection étant abaissé et le doigt en position rétractée, tandis que la figure 3 et la figure 4 sont des figures analogues aux figures 1 et 2, mais dans lesquelles les pompes sont activées, le capot de protection relevé et le doigt rétractable en position proéminente.

Les figures 5 et 6 sont des coupes horizontales des figures 1 et 3, détaillant une pompe individuelle, et le dispositif d'armement et de déclenchement des jets.

Les figures 7 et 8 représentent des coupes de la figure 1, détaillant le dispositif d'armement/déclenchement en position inactive et active.

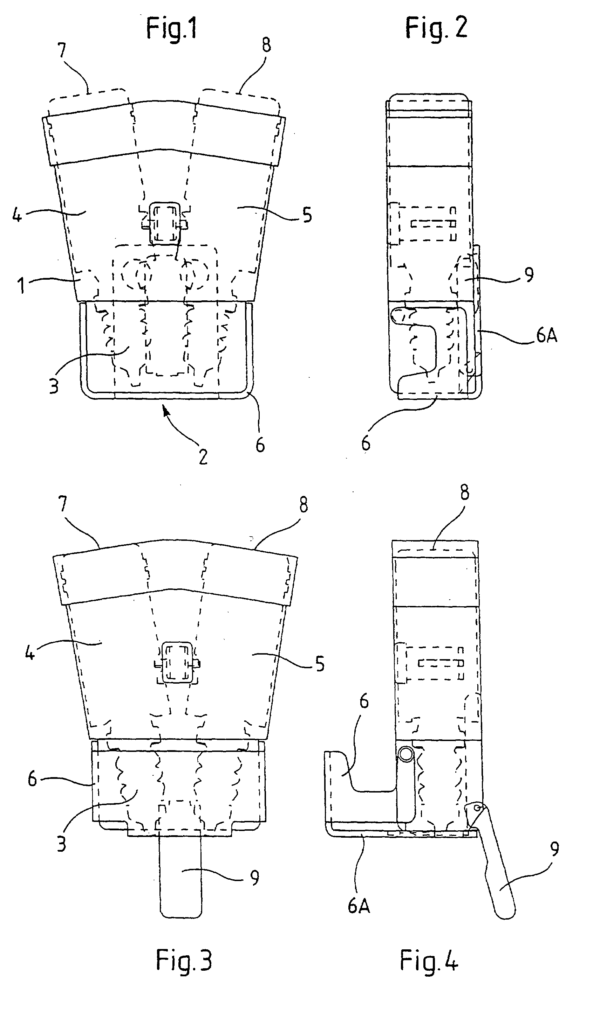

Sur la figure 1, on distingue le carter extérieur 1 comprenant à l'avant une large ouverture 2, donnant libre accès aux têtes 3 des pompes individuelles 4 et 5. Le capot de protection 6 abaissé permet de protéger et, le cas échéant, de cacher les têtes 3 des pompes 4, 5. On observe que les axes longitudinaux des pompes forment un "V", faisant converger les jets que lesdites pompes sont capables de projeter en un point situé à distance de l'avant du carter. Dans cette forme de réalisation, les extrémités arrière 7 et 8 des pompes 4 et 5 situées à l'arrière du carter, sont accessibles pour être par exemple pressées par un doigt pour passer en position activée.

En vue de leur armement simultané, on peut trouver à l'arrière du carter, et par exemple enfiché ou encliqueté sur celui-ci, un boîtier comprenant un bouton à presser guidé d'avant en arrière, par exemple par des glissières, et permettant l'activation simultanée des deux pompes ; on appuie donc à l'arrière des deux pompes simultanément par l'intermédiaire de ce bouton. Ainsi on peut éviter, si désiré, qu'une seule pompe soit armée avant son déclenchement.

Sur la figure 2, on distingue, vu de côté, le capot de protection des têtes des pompes 4 et 5, ainsi que le doigt 9 en position repliée ou escamoté. Le doigt 9 est caché par un plateau 6A, ici en position verticale, que comporte le capot.

Sur les figures 3 et 4, sur lesquelles les pompes sont en position activée, on peut observer que le capot 6 a été relevé, dégageant le doigt et les têtes 3 des pompes 4 et 5 car le plateau 6A comporte des évidements face aux têtes 3 des pompes. Le doigt 9 a été déplié afin de pouvoir abaisser la paupière. Enfin les arrières 7 et 8 des pompes 4 et 5 ont tous les deux été enfoncés, pour placer les pompes en position activée.

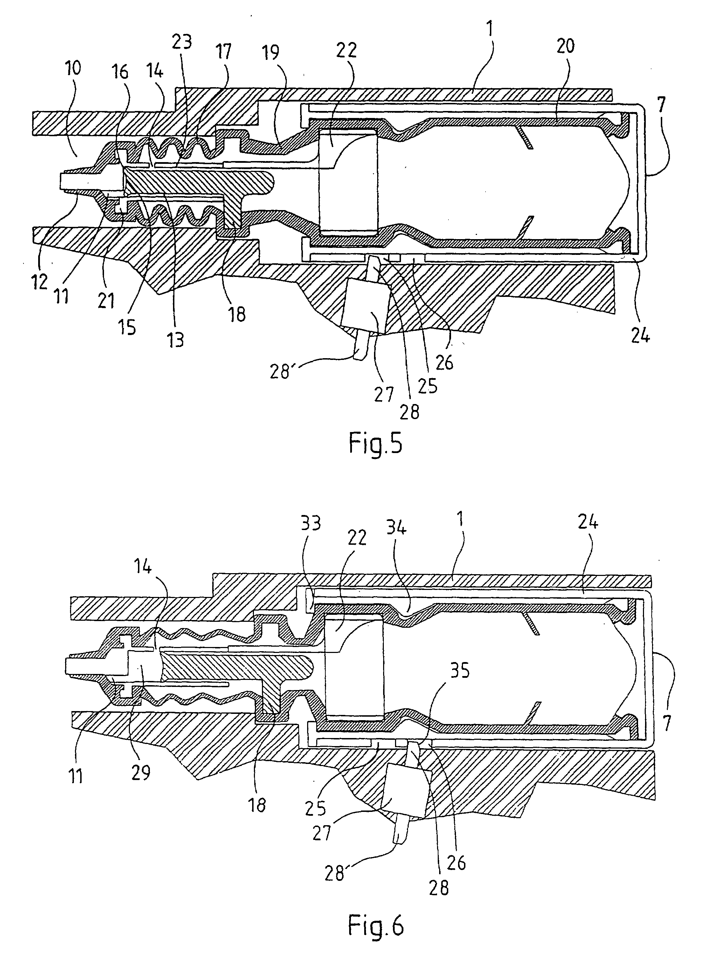

Sur la figure 5, on peut mieux observer le dispositif de déclenchement, ainsi qu'une pompe du type décrit dans FR-A-2725247. La pompe comprend un corps de pompe 23 ayant une extrémité antérieure (ou buse) du côté de la sortie du fluide 10 à l'avant du carter, ladite extrémité antérieure comportant un orifice de sortie 11, obturé par une membrane élastique 12 par exemple réalisée en Kraton® constituant l'avant du flacon. Le corps de pompe se poursuit vers l'arrière par un conduit de pompe 13 muni d'un orifice 14 latéral d'admission de fluide. Cette pompe comprend aussi un piston mobile installé dans le corps de pompe, le déplacement relatif de l'extrémité 15 du piston par rapport au corps de pompe 23 entre l'orifice 14 d'admission et une position en butée 16 située vers l'orifice de sortie 11 déterminant ainsi la quantité de fluide expulsé lors du déplacement relatif piston/corps de pompe. L'extrémité 15 du piston est ajustée hermétiquement à frottement doux au conduit de pompe 13. L'orifice d'admission 14 est de taille suffisante pour que seule la quantité de fluide prédéterminée puisse se trouver capturée dans l'extrémité du conduit de pompe 13 en vue de son expulsion par l'orifice de sortie 11. Le corps de pompe et le piston sont totalement enveloppés par le flacon 20 à l'exception de l'extrémité antérieure, c'est à dire la tête, du corps de pompe. On observe que l'orifice de sortie 11 est un canal dirigé axialement par rapport à la longueur de la pompe. Ainsi le liquide est expulsé dans l'axe longitudinal de la pompe. On observe que la pompe est pourvue d'un moyen élastique de rappel du piston en position de butée qui est constitué par le flacon lui-même. Il est d'ailleurs à noter qu'en fait ce n'est pas le piston, bloqué par l'anneau 18, qui se déplace, mais le corps de pompe maintenu vis à vis du flacon 20 à l'avant par l'anneau 21 et à l'arrière par l'anneau 22. Ce flacon est constitué d'une partie en accordéon 17 d'une épaisseur suffisante pour former un moyen de rappel acceptable. De l'autre côté de l'anneau 18 que comporte le piston, on observe un second moyen élastique de rappel 19 constitué par un épaississement de la paroi également pourvu d'une structure en accordéon. Dans cette réalisation, la membrane élastique 12 et le flacon élastique constituent une pièce unique 20. Le corps de pompe est cylindrique et comprend ici un anneau frontal 21 ainsi qu'un anneau postérieur 22. L'anneau frontal 21 ainsi que l'anneau postérieur 22 sont solidaires de l'extrémité d'une partie en accordéon du flacon élastique en s'engageant dans une rainure.

Plus particulièrement le piston a une forme allongée et est muni d'une pluralité d'ancres, à savoir des éléments radiaux, à l'extrémité desquelles se trouvent des éléments en forme d'arcs constituant l'anneau postérieur 18 du piston. Comme on l'a vu, le corps de pompe 23 est cylindrique et comprend un anneau frontal 21, un anneau postérieur cylindrique 22, de diamètre plus important que celui des arcs évoqués ci-dessus, le fond antérieur de l'anneau cylindrique postérieur étant évidé de telle sorte que les ancres ci-dessus puissent traverser le fond dudit anneau cylindrique et permettre ainsi par l'intermédiaire de rainures correspondant aux éléments radiaux ci-dessus et ménagés dans le cylindre constituant le corps de pompe 23, le déplacement longitudinal du piston. Ainsi, lorsque le piston recule, ce déplacement détermine une cavité dont l'état de pression est un vide partiel ; en effet l'orifice de sortie est bouché par la membrane élastique empêchant l'entrée de l'air dans la pompe. En reculant encore, le piston finit par arriver au niveau de l'orifice 14 de l'admission de fluide. A ce moment, le conduit de pompe se remplit rapidement du fluide contenu dans l'enveloppe 20 comme on le verra sur la figure 6. Le moyen de rappel 19 est alors compressé tandis que le moyen élastique de rappel 17 est en position étirée. Lorsque l'on déclenche l'éjection des jets, au moment où le piston arrive de nouveau au niveau de l'orifice d'admission de fluide 14 (en fait, c'est le corps de pompe qui se déplace relativement au carter alors que le piston reste bloqué), il emprisonne un volume prédéterminé de fluide qui est le volume qui est éjecté.

La pompe est contenue dans un boîtier 24 comportant deux encoches 25 à l'avant et 26 vers l'arrière. En position désarmée, une gâchette est introduite dans l'encoche avant 25. Sur cette vue de dessus, on distingue que la gâchette comprend un poussoir 27 muni de deux ailes 28 et 28'montées sur des languettes perpendiculaires au plan du dessin. L'aile 28' est destinée à s'introduire dans des encoches d'un autre boîtier d'une autre pompe qui n'est pas représentée sur cette figure.

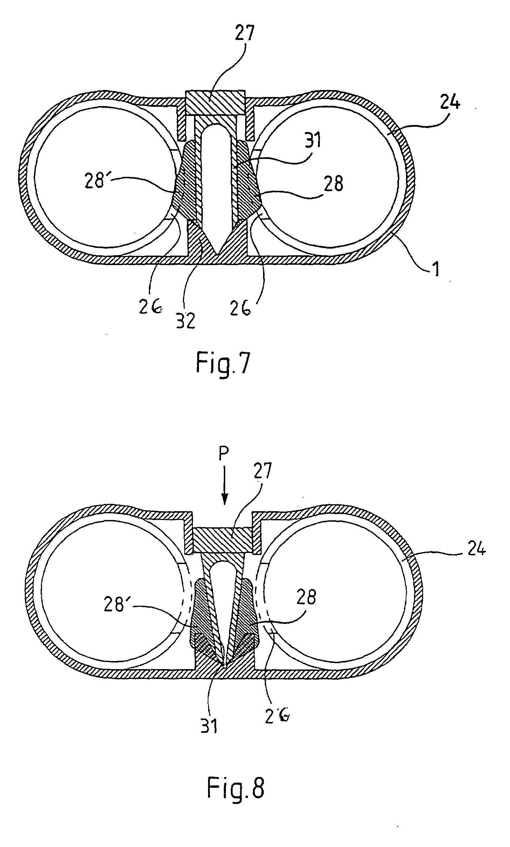

Sur la figure 6, on distingue les mêmes éléments que sur la figure 5, mais en position armée. On voit que dans cette position, le boîtier 24 a été avancé relativement au carter 1. Etant donné que le boîtier 24 maintient l'anneau postérieur 22 entre une butée de boîtier 33, et une nervure 34, et que l'avancée du piston est bloquée par la coopération de l'anneau 18 de piston avec un rétrécissement à l'avant du carter, l'enfoncement par pression sur l'extrémité arrière du boîtier contenant la pompe déplace le corps de pompe vers l'avant. De ce fait, il se crée une cavité 29 qui s'emplit de fluide par l'orifice d'admission 14. La forme biseautée de l'aile 28 grâce au biseau 35 a permis le passage de l'aile 28, de l'encoche avant 25 vers l'encoche arrière 26, bloquant le recul vis à vis du carter. Les extrémités libres des ailes sont capables de se rapprocher l'une de l'autre comme on le verra ci-après sur les figures 7 et 8. La pompe est ainsi en position armée. Si l'on appuie sur la gâchette 27, les ailes 28 et 28' s'escamotent et permettent au corps de la pompe de repartir vers l'arrière, comprimant ainsi la cavité 29. La pression ainsi constituée se communique à l'orifice 11 de sortie et permet d'écarter la membrane élastique 12 de l'extrémité du corps de la pompe, et ainsi d'éjecter la quantité de liquide prédéterminée et incluse dans la cavité 29.

Sur la figure 7, où les ailes 28 et 28' sont en position déployée, on observe que celles-ci sont introduites dans une encoche, par exemple 26, ménagée dans le boîtier 24 contenant le flacon 20. Ces ailes sont situées sur des languettes 31 que comporte le poussoir 27. Celles-ci peuvent coopérer avec un récepteur 32 en forme de "V" ménagé sur la base inférieure du carter. Ainsi une pression P sur le bouton poussoir 27, comme on le voit sur la figure 8, permet de rapprocher et d'escamoter les ailes 28 et 28' et ainsi relâcher le ou les boîtier(s) 24, permettant ainsi aux pompes de changer de position et éjecter les fluides.

On voit que le système ci-dessus préserve l'indépendance des flacons, permettant d'utiliser l'un ou l'autre, ou les deux simultanément.

Grâce à un tel distributeur, c'est la première fois que l'on peut administrer simultanément deux produits différents, en respectant leurs pH spécifiques tant pour la conservation que pour la pénétration intra oculaire.

C'est aussi la première fois que l'on peut également administrer une combinaison sans recourir à une reformulation de synthèse pour délivrer une goutte oculaire. C'est enfin la première fois que l'on peut administrer deux produits en une dose cumulée inférieure à 25 ml.

## Revendications

1. Distributeur double de liquides médicamenteux comprenant :
- un carter extérieur (1) comprenant au moins une ouverture avant (2) et une ouverture arrière,
- ledit carter contenant deux pompes individuelles (4,5) à déclenchement de jet jumelé, munies chacune d'un dispositif doseur délivrant des doses déterminées, la direction et le sens du jet projeté par lesdites pompes (4,5) par l'ouverture avant (2) du carter (1) convergeant vers un point situé au delà de l'extrémité avant du carter (1) selon un angle tel que l'écartement des jets soit prédéterminé, les deux jets quittant le distributeur par deux orifices d'éjection distincts,
- un dispositif de déclenchement (27) des jets, accessible depuis l'extérieur du carter (1).

2. Distributeur selon la revendication 1, **caractérisé en ce que** le dispositif de déclenchement (27) de la projection des jets est d'action simultanée.

3. Distributeur selon la revendication 1 ou 2, **caractérisé en ce que** les axes longitudinaux des pompes (4,5) déterminent la direction des jets.

4. Distributeur selon la revendication 1 ou 2, **caractérisé en ce que** les axes des têtes des pompes (4,5) déterminent la direction des jets tandis que les axes du reste des pompes (4,5) sont parallèles entre eux.

5. Distributeur selon l'une des revendications 1 à 4 **caractérisé en ce qu'**il comprend un doigt (9) proéminent à l'avant du carter (1).

6. Distributeur selon la revendication 5, **caractérisé en ce que** la direction et le sens du jet projeté par lesdites pompes (4,5) par l'ouverture avant du carter (1) convergent vers un point situé au delà de l'extrémité du doigt (9) écartée du carter (1), et au dessus de celui-ci selon un angle tel qu'au niveau de ladite extrémité, l'écartement des jets soit d'environ 3 à 5 mm.

7. Distributeur selon l'une des revendications 1 à 6, **caractérisé en ce que** les pompes sont contenues dans des boîtiers rigides (24) comportant des encoches (25, 26) latérales.

8. Distributeur selon l'une des revendications 2 à 7, **caractérisé en ce que** le dispositif de déclenchement (27) des jets est un bouton-poussoir comportant des languettes (31) munies de deux ailes (28, 28') perpendiculaires à ces languettes (31).

9. Distributeur selon la revendication 8, **caractérisé en ce que** les languettes coopèrent à leur extrémité opposée à la zone où s'effectue la pression pour déclencher les jets avec une fente en V ménagée sur le carter pour provoquer le rapprochement des languettes (31) et le désengagement des ailes (28, 28') des encoches latérales (25, 26) ménagées dans les boîtiers rigides (24) renfermant les pompes.

10. Distributeur selon l'une des revendications 1 à 9, **caractérisé en ce que** les pompes sont des pompes à armement préalable.

11. Distributeur selon l'une des revendications 1 à 10, **caractérisé en ce que** les pompes comprennent trois pièces, à savoir un corps de pompe (23), un piston et un flacon élastique (20) les renfermant, le corps de pompe ayant une extrémité antérieure du côté de la sortie de la pompe, ladite extrémité antérieure comportant un orifice (11) de sortie obturé par une membrane élastique (12) constituant l'avant du flacon et se poursuivant vers l'arrière par un conduit de pompe (13) muni d'un orifice (14) latéral d'admission de fluide, le piston étant installé dans ledit corps de pompe, le déplacement relatif de l'extrémité (15) du piston par rapport au corps de pompe (23) entre l'orifice d'admission et une position en butée (16) située vers l'orifice de sortie (11) déterminant ainsi la quantité de fluide expulsé lors du déplacement relatif piston/corps de pompe.

12. Distributeur selon la revendication 11, **caractérisé en ce que** le flacon comprend une première partie en accordéon (17) d'une épaisseur suffisante pour former un moyen de rappel acceptable et une seconde partie en accordéon (19), **en ce que** le piston comprend un anneau postérieur (18) bloqué entre les deux parties en accordéon (17, 19), et **en ce que** le corps de pompe est cylindrique et comprend un anneau frontal (21), solidaire de l'avant du flacon, ainsi qu'un anneau postérieur (22), solidaire de l'arrière du flacon derrière la seconde partie (19) en accordéon.

## Claims

1. Double dispenser for medicinal liquids, comprising:
- an outer casing (1) comprising at least one front opening (2) and one rear opening,
- the said casing containing two individual paired jet trigger pumps (4, 5), each provided with a dosing device dispensing predetermined doses, the aiming and direction of the jet projected by the said pumps (4, 5) through the front opening (2) of the casing (1) converging towards a point located beyond the front end of the casing (1) at an angle such that the separation of the jets is predetermined, the two jets leaving the dispenser through two separate ejection orifices,
- a trigger device (27) for the jets, accessible from the outside of the casing (1).

2. Dispenser according to Claim 1, **characterised in that** the trigger device (27) for the projection of the jets has simultaneous action.

3. Dispenser according to Claim 1 or 2, **characterised in that** the longitudinal axes of the pumps (4, 5) determine the direction of the jets.

4. Dispenser according to Claim 1 or 2, **characterised in that** the axes of the heads of the pumps (4, 5). determine the direction of the jets, while the axes of the remainder of the pumps (4, 5) are parallel to each other.

5. Dispenser according to one of Claims 1 to 4, **characterised in that** it comprises a protuberant finger (9) at the front of the casing (1).

6. Dispenser according to Claim 5, **characterised in that** the aiming and direction of the jet projected by the said pumps (4, 5) through the front opening of the casing (1) converge towards a point located beyond the end of the finger (9) furthest from the casing (1), and above the latter at an angle such that, at the level of the said end, the jets are separated by about 3 to 5 mm.

7. Dispenser according to one of Claims 1 to 6, **characterised in that** the pumps are contained in rigid cases (24) comprising lateral notches (25, 26).

8. Dispenser according to one of Claims 2 to 7, **characterised in that** the trigger device (27) for the jets is a push- button comprising tongues (31) provided with two wings (28, 28') perpendicular to these tongues (31).

9. Dispenser according to Claim 8, **characterised in that** the tongues cooperate, at their end opposite the zone in which the pressure is applied in order to trigger the jets, with a V- shaped slot formed in the casing in order to bring the tongues (31) together and release the wings (28, 28') from the lateral notches (25, 26) formed in the rigid cases (24) containing the pumps.

10. Dispenser according to one of Claims 1 to 9, **characterised in that** the pumps are pumps which can be primed in advance.

11. Dispenser according to one of Claims 1 to 10, **characterised in that** the pumps comprise three parts, namely a pump body (23), a piston and an elastic bottle (20) housing them, the pump body having a front end nearest the outlet of the pump, the said front end comprising an outlet orifice (11) obturated by an elastic membrane (12) constituting the front of the bottle and continuing towards the rear by a pump conduit (13) provided with a lateral fluid- inlet orifice (14), the piston being installed in the said pump body, the relative displacement of the end (15) of the piston with respect to the pump body (23) between the inlet orifice and a stop position (16) situated near the outlet orifice (11) thus determining the quantity of fluid expelled during the relative displacement between the piston and the pump body.

12. Dispenser according to Claim 11, **characterised in that** the bottle comprises a first bellows section (17) of sufficient thickness to form a satisfactory return means and a second bellows section (19), **in that** the piston comprises a rear ring (18) locked between the two bellows sections (17, 19), and **in that** the pump body is cylindrical and comprises a frontal ring (21), firmly attached to the front of the bottle, and a rear ring (22), firmly attached to the rear of the bottle behind the second bellows section (19).

## Patentansprüche

1. Doppelabgabevorrichtung für Arzneiflüssigkeiten, mit
- einem äußeren Gehäuse (1), das mindestens eine vordere (2) und eine hintere Öffnung aufweist,
- wobei das Gehäuse zwei einzelne Pumpen (4, 5) zum Auslösen eines Doppelstrahls umfaßt, deren jede mit einer Dosiervorrichtung versehen ist, die vorbestimmte Dosen abgibt, wobei Ausrichtung und Richtungssinn des von den Pumpen (4, 5) durch die vordere Öffnung (2) des Gehäuses (1) ausgestoßenen Strahls in einem Punkt, der hinter der vorderen Öffnung des Gehäuses (1) liegt, unter einem Winkel zusammenlaufen, bei dem der Abstand der Strahlen vorbestimmt ist, wobei die beiden Strahlen durch zwei unterschiedliche Ausstoßöffnungen aus der Abgabevorrichtung austreten,
- eine Vorrichtung (27) zum Auslösen der Strahlen, die von außerhalb des Gehäuses (1) zugänglich ist.

2. Abgabevorrichtung nach Anspruch 1, **dadurch gekennzeichnet, daß** die Vorrichtung (27) zum Auslösen des Ausstoßes der Strahlen gleichzeitig wirkt.

3. Abgabevorrichtung nach Anspruch 1 oder 2, **dadurch gekennzeichnet, daß** die Längsachsen der Pumpen (4, 5) die Richtung der Strahlen bestimmen.

4. Abgabevorrichtung nach Anspruch 1 oder 2, **dadurch gekennzeichnet, daß** die Achsen der Pumpenköpfe (4, 5) die Richtung der Strahlen bestimmen, während die Achsen des Restes der Pumpen (4, 5) parallel zueinander verlaufen.

5. Abgabevorrichtung nach einem der Ansprüche 1 bis 4, **dadurch gekennzeichnet, daß** sie einen vorne am Gehäuse (1) vorstehenden Finger (9) umfaßt.

6. Abgabevorrichtung nach Anspruch 5, **dadurch gekennzeichnet, daß** Ausrichtung und Richtungssinn des von den Pumpen (4, 5) durch die vordere Öffnung des Gehäuses (1) ausgestoßenen Strahls in einem Punkt hinter dem vom Gehäuse (1) entfernten Ende des Fingers (9), und über diesem, unter einem Winkel zusammenlaufen, bei dem der Abstand der Strahlen an diesem Ende etwa 3 bis 5 mm beträgt.

7. Abgabevorrichtung nach einem der Ansprüche 1 bis 6, **dadurch gekennzeichnet, daß** die Pumpen in steifen Gehäusen (24) enthalten sind, die seitliche Aussparungen (25, 26) umfassen.

8. Abgabevorrichtung nach einem der Ansprüche 2 bis 7, **dadurch gekennzeichnet, daß** die Vorrichtung (27) zum Auslösen der Strahlen ein Druckknopf ist, der Zungen (31) aufweist, welche mit zwei senkrecht zu diesen Zungen (31) verlaufenden Flügeln (28, 28') versehen sind.

9. Abgabevorrichtung nach Anspruch 8, **dadurch gekennzeichnet, daß** die Zungen an ihrem Ende gegenüber dem Bereich, in dem die Druckbetätigung zum Auslösen der Strahlen erfolgt, in Wirkverbindung mit einer V-Nut stehen, die an dem Gehäuse ausgebildet ist, um das Zusammenführen der Zungen (31) und das Lösen der Flügel (28, 28') aus den seitlichen Aussparungen (25, 26), die in den die Pumpen umgebenden, steifen Gehäusen (24) ausgebildet sind, zu bewirken.

10. Abgabevorrichtung nach einem der Ansprüche 1 bis 9, **dadurch gekennzeichnet, daß** die Pumpen vorgefüllte Pumpen sind.

11. Abgabevorrichtung nach einem der Ansprüche 1 bis 10, **dadurch gekennzeichnet, daß** die Pumpen drei Teile umfassen, nämlich einen Pumpenkörper (23), einen Kolben und einen elastischen Flakon (20), der sie umgibt, wobei der Pumpenkörper ein vorderes Ende auf der Seite des Ausgangs der Pumpe aufweist und dieses vordere Ende eine Auslaßöffnung (11) umfaßt, die mit einer elastischen Membran (12) verschlossen ist, die den Vorderteil des Flakons bildet und sich nach hinten in einem Pumpenkanal (13) fortsetzt, der mit einer seitlichen Fluideinlaßöffnung (14) versehen ist, wobei der Kolben im Pumpenkörper angebracht ist und die Relativverschiebung des Endes (15) des Kolbens relativ zum Pumpenkörper (23) zwischen der Einlaßöffnung und einer Anschlagstellung (16), die zur Auslaßöffnung (11) hin liegt, dabei die Fluidmenge bestimmt, die bei der Relativverschiebung von Kolben/Pumpenkörper ausgestoßen wird.

12. Abgabevorrichtung nach Anspruch 11, **dadurch gekennzeichnet, daß** der Flakon einen ersten ziehharmonikaförmigen Teil (17) mit einer Dicke, die ausreicht, um ein akzeptables Rückstellmittel zu bilden, und einen zweiten Ziehharmonika-Teil (19) umfaßt, daß der Kolben einen hinteren Ring (18) aufweist, der zwischen den beiden Ziehharmonika-Teilen (17, 19) arretiert ist, und daß der Pumpenkörper zylinderförmig ausgebildet ist und einen vorderen Ring (21), der fest mit dem Vorderteil des Flakons verbunden ist, sowie einen hinteren Ring (22) aufweist, der fest mit der Rückseite des Flakons hinter dem zweiten Ziehharmonika-Teil (19) verbunden ist.
